Europäisches Patentamt

European Patent Office

Office européen des brevets

(19)

(11) EP 1 154 272 A2

## (12) EUROPEAN PATENT APPLICATION

(43) Date of publication:
14.11.2001 Bulletin 2001/46

(51) Int Cl.$^7$: G01N 33/36, G06F 17/00

(21) Application number: 01660091.8

(22) Date of filing: 07.05.2001

(84) Designated Contracting States:
AT BE CH CY DE DK ES FI FR GB GR IE IT LI LU
MC NL PT SE TR
Designated Extension States:
AL LT LV MK RO SI

(30) Priority: 08.05.2000 FI 20001076

(71) Applicants:
• Berg, Carl-Gustav
  20100 Turku (FI)
• Akerholm, Johan
  20740 Turku (FI)

(72) Inventors:
• Berg, Carl-Gustav
  20100 Turku (FI)
• Akerholm, Johan
  20740 Turku (FI)

(74) Representative: Heikkilä, Hannes Antero
Turun Patenttitoimisto Oy,
P.O. Box 99
20521 Turku (FI)

(54) **System and method for determining quantities in a coating process, method for controlling a drying process and computer program product**

(57) The invention relates to a system and a method, whereby laboratory measurements can be used for determining dynamic changes in the properties of a coating material and/or a material (2) to be coated, as a liquid-containing coating material is applied onto the material (2) to be coated. The essential quantities in terms of drying technology can be calculated by means of measuring results obtained through the invention and by means of per se known algorithms by using optimization. This optimization can be performed by means of a computer program product built for this purpose. Obtained results are used for controlling an industrial drying process.

EP 1 154 272 A2

**Description**

The invention relates to a system and a method according to in the preambles of the appended independent claims for determining quantities of a coating process, a method for controlling a drying process, as well as a computer program product for the exploitation of results obtained by means of said system and said methods. The invention enables laboratory measurements to be used more effectively than heretofore for the determination of dynamics involved in a coating process, and for predicting the progress of a coating process in production.

The literature references cited in this text must be regarded as part of the prior art, both in terms of theory discussed therein in relation to the present invention, and particularly in terms of examples described therein.

At present, the laboratory measurements for coating process quantities are generally based on separate measurements for either a material to be coated, such as paper, or for a coating material, such as a coating mix. It is also known to perform coating tests in laboratory or by means of a pilot machine, thereby studying the properties of a finished coated product, such as paper. However, testing of individual materials may be a far cry from the condition in real-life production, and does not provide a reliable conception about the progress of a coating process. Testing processes for finished coated materials are carried out after a certain time lapse from the actual time of coating, the possibility of studying the first incidents of a coating process being already lost. For example, the drying of materials and the migration of various ingredients in a coating material, such as a coating mix, commence immediately as the material to be coated and the coating material come to contact. The gradients of quantities representing dynamics are generally at their peak in the early stages of a process, hence the variations in quantities also being often at the maximum speed thereof.

For example, the paper coating process with modern high-speed coating heads is so fast that even during the first second major changes takes place in the properties of paper and a coating material, such as temperatures as well as concentrations of water and other substances. Examination of these changes has not been possible until now. Good knowledge about the early stages of a coating process is, to say the least, a necessary indicator for a more precise prediction regarding the entire coating process.

In literature [Letzelter, 1993], a number of different measuring methods has been disclosed for studying properties of coated paper, for example. The known measuring methods are mostly based on measuring the viscosity of a coating mix and on measurements for the sorption ability of paper. One currently employed testing method for quantities of coated paper is so-called ÅA-GWR [Letzelter, 1993]. However, this method is not capable of examining high-speed dynamics of a coating process, nor is it capable of studying a genuine combination of base paper-coating mix. The ÅA-GWR method can be used for revealing the static behaviour of a coating material. The changes taken place in paper or a coating material cannot be found out individually. In current measuring methods, the measuring data is often on a wrong scale, in ÅA-GWR, for example, the transfer of water may obtain a value of for example 100 $g/m^2$, while the correct value would be for example 5 $g/m^2$. The measuring times of known methods are generally other than truthful, the ÅA-GWR, for example, using a measuring time of about two minutes, while in reality the process takes place in about two seconds.

It is known [Heikkilä, 1993] to manually pick up samples from a pilot machine with sort of knives, to weigh the samples, and to draw conclusions therefrom regarding the progress of drying. However, the results of such tests have exhibited poor reproducibility, and hence the conclusions that can be drawn therefrom have poor reliability. The poor quality of reproducibility in the use of a pilot machine, regarding precise measurements of water sorption and measurements of other liquid substances, is most of the time due to the fact that the physical state of base paper cannot be continuously adjusted with the same accuracy as in laboratory. In a continuous-action pilot machine, it is also impossible to determine accurately the physical condition of base paper and the physical condition of the surface of base paper. A result of this is that, in the process of coating with a pilot machine there is no accurate knowledge regarding thermal and moisture gradients of paper, aspects that are controllable in laboratory conditions. When samples are picked up from a pilot machine or a full-scale machine, the sampling process is extremely costly.

Patent literature discloses several methods for studying a paper coating. For example, Finnish patent FI91025 describes a method and apparatus applicable in the coating head of a paper machine for adjusting the amount of coating on a paper web in terms of its cross-sectional profile.

US patent 2353852 discloses an apparatus for a visual examination of substances absorbing in paper during a coating process. The apparatus is not intended for the absolute survey of physical quantities, but for visual examination only. The apparatus of US publication 2353852 is used for coating paper with a pigment-containing coating material and extra coating is immediately removed by suction. The change of colour in paper indicates visually how much pigment had time to be absorbed. This method is not capable of measuring any kind of numerical values. In addition, this method can only be used for studying the transfer of a given pigment from coating to paper, the movement of liquids in the coating itself is not examined.

It is an object of the present invention to provide such a system, methods, and computer program product, which are capable of minimizing the above-mentioned problems appearing in the prior art.

The foregoing drawbacks can be eliminated or alleviated and the above objects can be accomplished with a system, methods, and a computer program product of the invention, which are characterized by what is set forth in the characterizing clauses of the appended claims.

The present invention is particularly suitable for use in paper industry, especially for studying a coating process effected with a paste, but also for studying the lamination of paper. The invention is also applicable to studies regarding the setting of a paper printing ink, such as for example in the process of studying the movement of water or solvents of offset ink. The invention can also be exploited in some other industry, such as in construction industry for studying mortar drying on the surface of a brick or the like, or in paint industry for studying a paint drying process, for example for surveying the drying process when painting wood or metal. It is the central idea of the invention that laboratory measurements are capable of determining dynamic changes in the properties of a coating material, such as a coating slip or mix, and/or a material to be coated, such as paper, as the coating material, containing liquid, such as water, a solvent or the like, is laid on the material to be coated. For example, when an aqueous coating mix and porous and/or hygroscopic paper to be coated come to contact with each other, the water starts to move between the coating mix and the paper. The movement may occur just as well in aqueous phase as in vapour phase. The invention provides measuring results which reveal the dynamics of these movements, which subsequently in this text is referred to as drying of a coating material. Certain essential quantities involved in drying technology can be calculated by means of measuring results obtained through the invention and by means of per se known algorithms by using optimization. This optimization can be performed by means of a purpose-built computer program product, the algorithms applied therein taking into account also changing ambient conditions, for example the moisture and temperature of paper and air. Thus, it is possible to come up with a product-specific prediction regarding the progressive drying of a coating slip even in conditions essentially unlike those prevailing at the time of measurement. In addition to the above, it is possible to come up with a product-specific prediction regarding the behaviour of a coated product in industrial drying processes. The examples of this text describe exemplary methods, systems, and computer program products, as well as the application thereof in paper industry as paper is coated with an aqueous coating slip. The invention is just as well applicable to other coating processes, for analyzing, predicting, and controlling the same.

It is one of the most important benefits of the invention that it offers exact information regarding dynamics in the early stages of a coating process. A good knowledge thereof, along with current knowledge and information technology regarding the modelling of physical processes, enables the entire coating process to be predicted and controlled in a new and more accurate manner. The invention is also capable of providing other information about the interaction between a coating and a material to be coated, and about a drying process in general. For example, it is possible to study the immobilization or solidification point of a coating mix and to obtain information regarding the surface roughness of base paper. A particularly significant benefit gained by the invention is that several of the forecited aspects are accessible for a new type of reliable information with a single measuring method.

Yet another especially important benefit is that the measuring results obtained by means of a method of the invention can be used for predicting a drying process in a reliable fashion even in conditions essentially different from measuring conditions. Said predictions are readily and accurately obtainable by means of computer program products according to the invention.

The invention will now be described in more detail with reference made to the accompanying schematic drawings and formulae, wherein

Fig. 1 shows schematically in a plan view a system of the invention for determining quantities of a coating process,
Fig. 2 shows the system of Fig. 1 in a view from behind,
Fig. 3 shows the system of figs. 1 and 2 in a frontal view,
Fig. 4 shows a sample knife of the invention in a frontal view,
Fig. 5 shows the sample knife of Fig. 4 in a side view,
Fig. 6 shows the operation of a method and a computer program product of the invention in the way of a flow chart,
Fig. 7 shows schematically the texture of a coated paper,
Fig. 8 shows by way of example a way of dividing a coated paper into layers required in mathematical models,
Fig. 9 shows the amount of water absorbed from coating mix into coated paper, measured and simulated at two different temperatures in a laboratory test conducted with methods of the invention, and
Fig. 10 shows data calculated with a method of the invention for a pilot-scale paper coating machine.

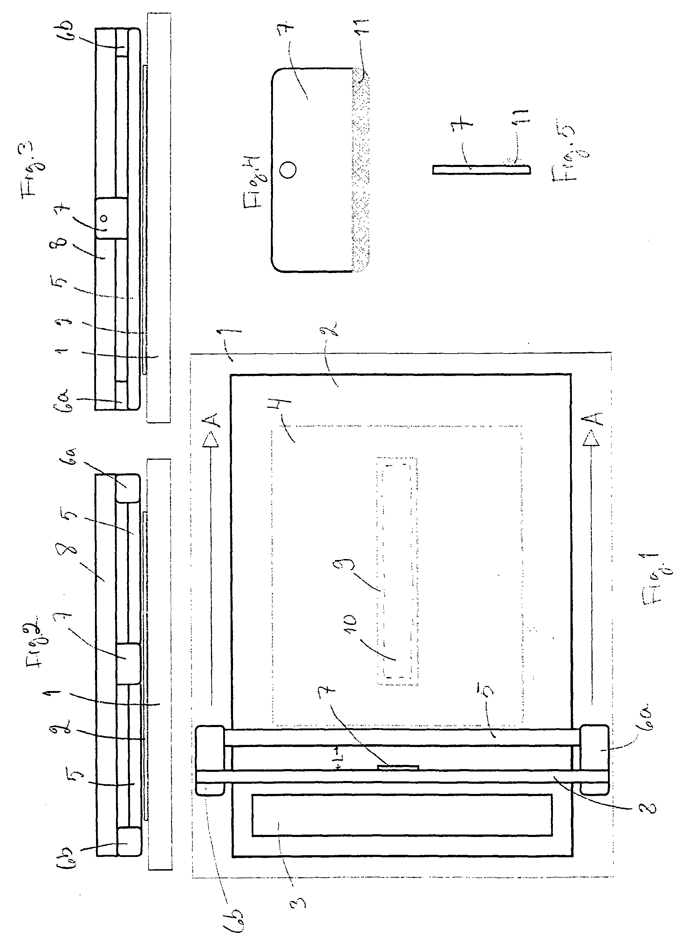

Figs. 1, 2, 3, 4 and 5 illustrate a system of the invention for determining quantities of a paper coating process, which enables the implementation of a method of the invention for determining the coating process quantities. The system, in terms of its configuration, operation, and application, as well as the method, in terms of its processes, will now be described in view of reference numerals presented in said Figures.

The inventive system comprises a foundation 1, upon which is laid a base paper 2 to be coated with a coating slip. The foundation 1 can be for example a table, a silicone mat or some other appropriate bed, to which the paper 2 can be conveniently bonded. The foundation may be flat, but just as well the inventive foundation can be in a curved or cylindrical shape or in another suitable shape. The paper 2 is preferably left without a coating over an area 3, which can be cut off of the base paper 2. Thus, the same paper sheet 2 can be used for an uncoated reference paper and a coated paper. The piece of paper 3 can be used, for example, to determine grammage $(g/m^2)$ for the base paper 2 by dividing the weight of the piece of paper 3 with the surface area of the piece of paper 3. The grammage of coated paper is always determined in dry state, in other words the paper must be most preferably dried at more than $105°C$ prior to its weighing. The coating of paper is effected by means of a coating device 5, which can be for example a steel bar, a coating blade, or a spray coater. For example, the bar or blade coater include in itself some sort of knife or doctor for smoothing the coating over a region to be coated. The coating device 5 is carried across the base paper 2 by means of guide elements 6a and 6b connected with the ends of the coating device 5. The guide elements 6a and 6b of the coating device are also fitted with a bracket 8 of a sample knife 7. The guide elements 6a and 6b are adapted to travel in a direction of application A for the base paper 2 along rails (not shown in the Figures) fixed to the foundation 1, whereby the sample knife 7, accompanied by its bracket 8, as well as the coating device 5 also travel in the same direction A across the base paper 2. The guide elements 6a and 6b and the rails can be replaced with another suitable arrangement. There may be a number of sample knives 7 in parallel and/or succession in the direction of application. The coating device 5 can be provided with its own supply bin for a coating mix, or else coating mix is supplied to the device 5 by pouring or along a tube, a hose or the like.

As the coating device 5 is driven across the paper sheet 2, a section 4 thereof will be coated. When the sample knife 7 is positioned above the coated region 4, said sample knife 7 is pressed, by means of the bracket 8, against the coated paper 4 in such a way that some of the coating mix will be scraped off an area 9. After the coating and doctoring, the area 9 is cut to remove a piece of paper 10 (shown in dashed line in Fig. 1) from the base paper 2, and grammage for the piece of paper 10 will be determined the same above-described way as that of the piece of paper 3. The piece of paper 3 and the piece of paper 10 are preferably equal in area for easier calculation.

The sample knife 7 is weighed prior to coating and doctoring. As soon as the coating is doctored off the surface 4, the sample knife 7 will be weighed together with a coating mix 11 adhered thereto. After the sample knife 7 and the scraped-off coating mix 11 have been weighed, said coating mix 11 present on the sample knife 7 will be dried. After the coating 11 has dried, it will be re-weighed together with the sample knife 7. The discrepancy between these two weighings can be used for determining the dry content of the scraped-off coating mix 11 at the time of doctoring.

After the grammage of the piece of paper 3, the grammage of the piece of paper 10, and the mass of the mix 11 adhered to the sample knife 7 have been determined, it is possible to determine an amount of coating present on the piece of paper 10. This proceeds as follows: the mass of the doctored mix 11 is first divided by the area $(g/m^2)$ of the piece of paper 10, followed by adding thereto the grammage $(g/m^2)$ of the piece of paper 10. From this total is subtracted the grammage of the piece of paper 3, and the result will be the amount of coating $(g/m^2)$ on the piece of paper 10.

Since the dry content of a coating mix prior to its application to the base paper 2 can be readily established, and since the dry content of the doctored mix and the amount of coating on the piece of paper 10 have been determined as described above, it will now be possible to calculate a change in the coating mix dry content during the interval between application and doctoring, i.e. the amount of liquid which has migrated from the coating mix into the base paper 2 as per area $(g/m^2)$.

The coating device 5 can be driven across the paper 2 at various speeds and, furthermore, a distance L between the coating device 5 and the sample knife 7 can be set as desired. This enables an adjustment of the time period between application and doctoring. Thus, this method enables the determination of the drying of a coating mix as a function of time lapsed between application and doctoring of the coating. The inventive system makes it possible to doctor a coating at the same spot two or more times, thus enabling the coating properties to be measured in various layers. The direction of application A is preferably essentially straight for facilitating an accurate establishment of the time period between application and sampling. Naturally, the coating can be applied and doctored in ways other than evenly and linearly.

In the example of figs. 1-5, the material to be coated is stationary and the coating device 5 as well as the sample knife 7 are moved. The system and method of the invention are equally functional in such a way that the material to be coated, for example a paper web, is carried past a stationary coating device 5 and sample knife 7.

Hence, the inventive sample knife 7 according to the invention is used for scraping off at least some of the coating. The coating device 5 in itself may also include some sort of doctor element. For example, when a coating is applied with a bar or blade coater, the coating material will be smoothed on the surface of a material to be coated by means of a sort of scraper. However, the inventive sample knife 7 has a totally different function, i.e. to disrupt a coating layer produced by the coating device 5 for picking up a sample 11. According to the invention, the sample knife 7 can also be provided as part of a smoothing blade included in the coating device 5 itself. It is preferred, however, that the sample knife 7 be separate from the blade of the coating device 5.

In order to facilitate the investigation of the sample 11 picked up by the sample knife 7, it is preferred that the sample knife 7 be readily removable from its carrier. It is possible to provide the sample knife 7 with elements, such as electrical sensors, for investigating a sample directly on the sample knife 7 without having to remove the sample knife 7 from the rest of the assembly.

Other measuring methods are also available for determining the water content of a coating mix applied to a base paper. For example, the water content of a coating can be established by means of an infrared (IR) sensor. However, in order to determine the amount of water escaped from a coating, it is necessary to measure also the amount of coating over the same area which is used for measuring the water content. By means of the inventive measuring method, both of these measurements can be readily implemented. Moreover, the currently available IR gauges are not able to determine the distribution of water content in a coating and a base and, thus, the measuring data is not sufficiently accurate. Another prior art way of measuring moisture is to measure the same conductively, i.e. by measuring a moist material for its capacitance or its resistive properties and to calculate the moisture content on the basis of thus obtained values. The development of laboratory-scale electrical measuring equipment is continuous and facilitates measurements for the electrical properties of materials in a totally new way. The electrical measurement of moisture or dry content enables a more effective automation of the method. In case the measurements are implemented in continuous action, i.e. on-line, the measuring operation will be enhanced even further.

On the basis of conducted tests, the inventive system and method for determining quantities of a paper coating process are recommended for the following values:

- recommended area for base paper 2 is in the order of A5...A1,
- recommended speed v for coating device 5 at the time of application is 0...100 cm/s,
- recommended distance L for doctoring off a coating is 0.1...10 cm,
- recommended drying temperature in all drying stages is about 5°C in excess of solvent boiling point. In case of water, this requires using of more than 105°C at a standard pressure of 101.325 kPa,
- recommended contact time for base paper and coating, which is calculated by dividing distance L with coating device speed v, is 0...3 s,
- measuring points should be made with 3...10 different contact times in order to establish transfer of water as a function of time,
- each contact time should be used for 2...10 tests in order to obtain a reliable average for measuring data,
- it is recommended that all tests in a single test series be conducted in equal external conditions, in other words, at equal temperature and at equal dry content for paper as well as coating mix,
- if coating is poured onto an absorbent material prior to spreading a coating, the application is recommended to be made on the surface of a water-transfer inhibiting medium, such as a plastic film. This serves to oppose a change of dry content prior to actual measuring process.

The inventive method and/or system for determining quantities of a coating process can be used for obtaining information about a paper coating process regarding at least the following quantities: the dynamic water retention of a coating, the dynamic sorption and desorption of a paper, the surface roughness of a paper, and the critical point of a coating.

The measuring data, which is obtained by means of the above-described system and/or method for determining quantities of a coating process, can be exploited for more information about a coating process than just those values measured at the measuring points. Said measuring data and known physical models, for example those representing a drying process, can be used for a mathematical optimization of values for product-specific quantities, such as a diffusion coefficient D or a subsequently described set of coefficients $\varepsilon\psi\eta$, outside the conditions existing at measuring points. This enables a high precision prediction regarding e.g. the following matters:

- drying dynamics of a coating at temperatures substantially different from those experienced during tests,
- drying dynamics of a coating at coating dry contents substantially different from those experienced during tests,
- drying dynamics of a coating in external conditions, such as evaporation intensity, air temperature, air moisture content, and thermal conductivity, which are substantially different from conditions during tests,
- sorption and desorption dynamics of a material to be coated in conditions substantially different from those experienced during tests.

The above aspects offer a possibility of working out product-specific drying calculations for example for the drying section of a paper coating machine or for a coated or painted wooden surface.

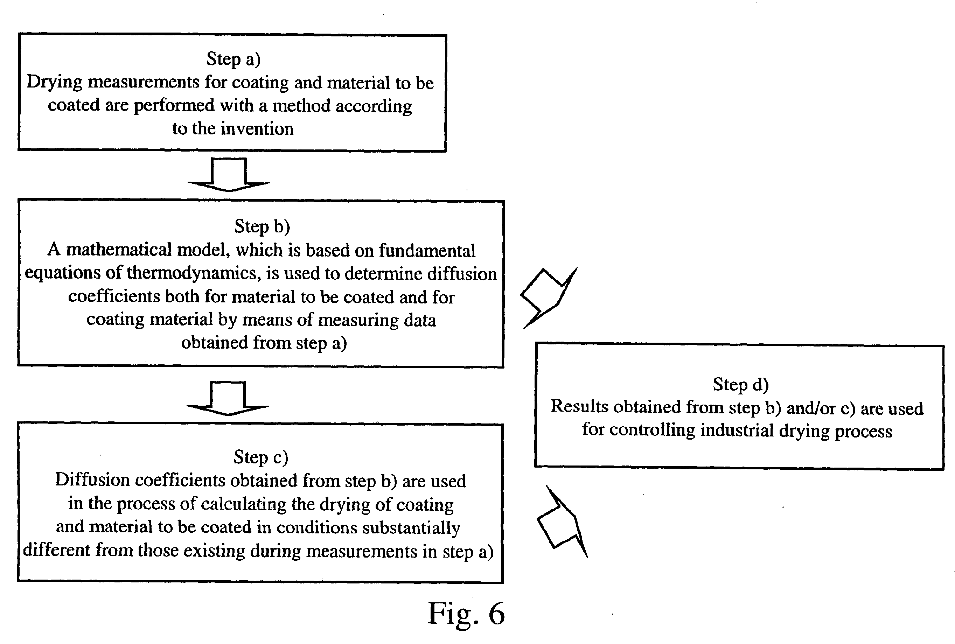

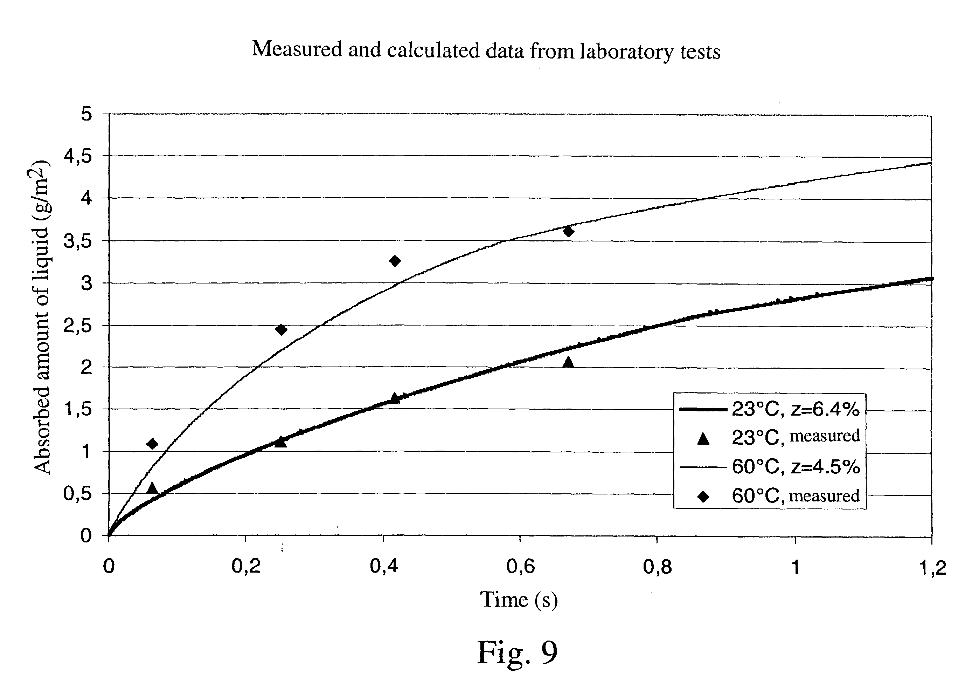

Fig. 6 shows a flow chart representing the operation of a method according to the invention for controlling a drying process, as well as the operation of a computer program product. This method and computer program product make use of values obtained by means of a method and/or system according to the invention for determining quantities of a coating process. In step a), the above-described method is used for drying measurements for a coating and/or a material to be coated, i.e. for determining desired quantities of a coating process. Results obtained experimentally from step a) are shown in the way of dots in Fig. 9.

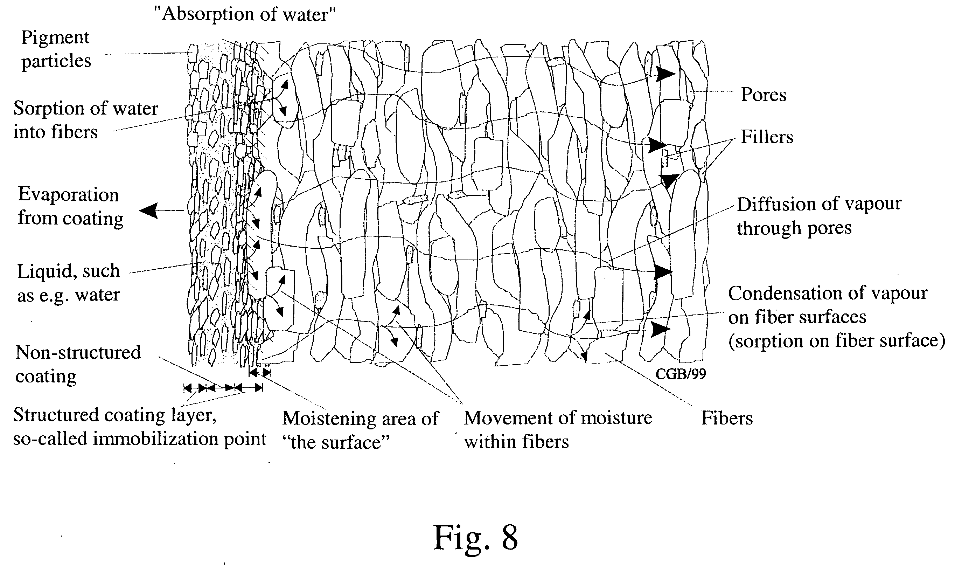

In step b), a mathematical model based on fundamental equations of thermodynamics are used for establishing diffusion coefficients both for a material to be coated and a coating material by exploiting the measuring data obtained from step a). Such a model and its application are shown in Fig. 8 and in equations 1-7.

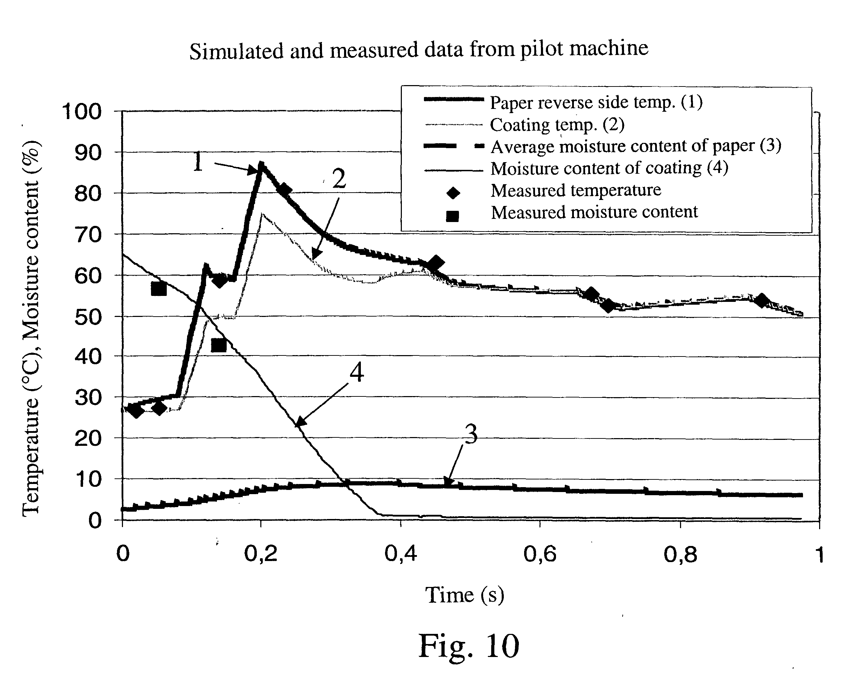

In step c), the diffusion coefficients, matched to measuring data and obtained from step b), are exploited by working out situations, wherein conditions differ substantially from those experienced during measuring operations. Thus, in step c), another per se known mathematical model is used for working out a drying process, at a temperature other than that used for example in the tests of step a) or for example in an industrial paper coating process. The exemplary results obtained from step c) are shown graphically in Figs. 9 and 10.

In step d), the results obtained from step b) and/or c) are used for controlling an industrial coating process. The inventive method is capable of providing an accurate prediction regarding optimal operating conditions for example in the drying section of a paper coating machine, without expensive investments in on-line measuring equipment.

Typically, the inventive method is implemented by means of a computer program product, which is directly uploadable in the central memory of a computer, or by means of a computer program product, which is stored in an element readable by a computer, said computer program product containing program code elements for performing a method of the invention while running said computer program product in a computer. In this context, the computer program product refers to an independent computer software or a computer software segment, which may comprise one or more program code elements. The program code element refers to an element consisting of one or more instructions or commands in a mode readable by a computer. The element readable by a computer refers in this context to all such means which can be used for a permanent or temporary storage of information, instructions, commands, instruction or command sequences, or other such material readable by a computer. Such means include for example hard disks, mass storages, buffer storages, diskettes, and cd-rom disks.

A typical computer program product of the invention comprises program code elements automatically performing the calculations of steps b), c), and d) after the results obtained from step a) are input to the program. In addition, the computer program product comprises a program code element, which supplies its operator or supervisor with values for controlling an industrial coating process on the basis thereof. The computer program product may also comprise a program code element capable of an automated adjustment of process quantities, such as the performance of heaters. Step a) may also be automated in such a way that the results obtained therefrom are uploaded automatically into the computer program product.

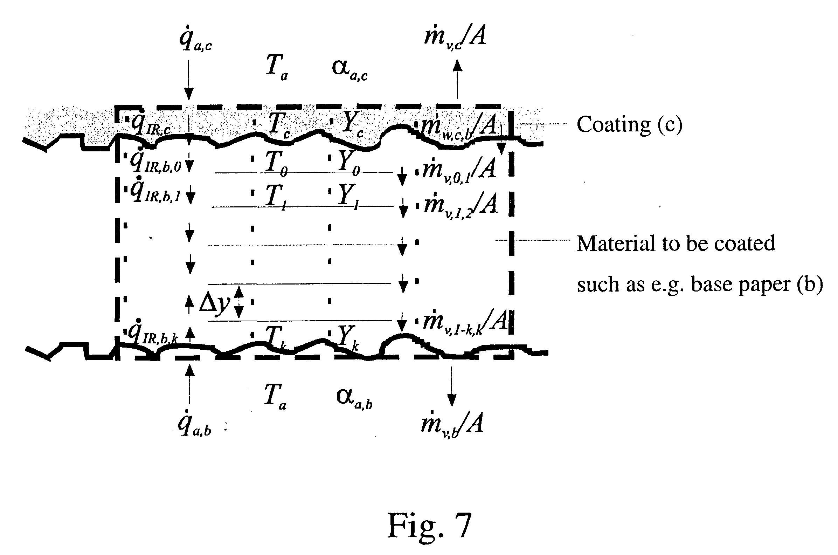

Fig. 7 shows the texture of a coated paper, as well as the movements of water as paper is being coated. As shown in the Figure, the coating dries as water evaporates in air, as water transfers in the form of vapour diffusion into a base paper, and along with surface moistening of paper. Moisture travels in the base paper as vapour diffusion until the base paper has a moisture content sufficiently high to go beyond a hygroscopic range, i.e. the moisture content is higher than 0.2 - 0.3 kg $H_2O$/kgbd, depending on a grade of paper. Water vapour condenses on the surfaces of fibers, from which it absorbs into the fibers.

Fig. 8 shows a way of dividing a coated paper into layers for facilitating mathematical modelling. The same method can be applied for modelling any other material to be coated and any other coating. The following differential equations employ the notations of Fig. 8. Around each stratum or element it is possible to calculate mass and energy balances. Equations 1 a-d represent these mass balances and equations 2 a-d represent the respective energy balances. Fig. 8 also illustrates index abbreviations for quantities in equation sets 1 and 2. This enables the calculation of temperature and moisture content for a coating and a base paper in the z-direction along a coated paper.

$$\frac{m_c}{A}\frac{dY_c}{d\tau} = -\frac{\dot{m}_{v,c,a}}{A} - \frac{\dot{m}_{w,c,b}}{A} \tag{1 a}$$

$$\frac{m_b}{kA}\frac{dY_{b,0}}{d\tau} = +\frac{\dot{m}_{v,b,c}}{A} - \frac{\dot{m}_{v,0,1}}{A} + \frac{\dot{m}_{w,c,b}}{A} \tag{1 b}$$

$$\frac{m_b}{kA}\frac{dY_{b,n}}{d\tau} = +\frac{\dot{m}_{v,n-1,n}}{A} - \frac{\dot{m}_{v,n,n+1}}{A} \tag{1 c}$$

$$\frac{m_b}{kA}\frac{dY_{b,k}}{d\tau} = -\frac{\dot{m}_{v,b,a}}{A} + \frac{\dot{m}_{v,n,k}}{A} \tag{1 d}$$

$$c_{p,c}\frac{m_c}{A}\frac{dT_c}{d\tau} = \alpha_{a,c}(T_a - T_c) - \frac{2\cdot\lambda_b}{\Delta y}(T_c - T_0) + \frac{\dot{q}_{IR,c}}{A} - \frac{\dot{q}_{v,c,a}}{A} \tag{2 a}$$

$$\rho_b \cdot c_{p,b} \cdot \Delta y_b \frac{dT_0}{d\tau} = \frac{2\cdot\lambda_b}{\Delta y}(T_c - T_0) - \frac{\lambda_b}{\Delta y}(T_0 - T_1) + \frac{\dot{q}_{IR,b}}{kA} - \frac{\dot{q}_{v,b,c}}{A} - \frac{\dot{m}_{v,0,1}}{A}\Delta h_v\{T_0\} \tag{2 b}$$

$$\rho_b \cdot c_{p,b} \cdot \Delta y_b \frac{dT_n}{d\tau} = \frac{\lambda_b}{\Delta y}(T_{n-1} - T_n) - \frac{\lambda_b}{\Delta y}(T_n - T_{n+1}) + \frac{\dot{q}_{IR,b}}{kA} - \frac{\dot{m}_{v,n,n+1}}{A}\Delta h_v(T_n) + \frac{\dot{m}_{v,n-1,n}}{A}\Delta h_v\{T_n\} \tag{2 c}$$

$$\rho_b \cdot c_{p,b} \cdot \Delta y_b \frac{dT_k}{d\tau} = \frac{\lambda_b}{\Delta y}(T_{k-1} - T_k) - \frac{\alpha_{a,b}\dfrac{2\cdot\lambda_b}{\Delta y}}{\alpha_{a,b} + \dfrac{2\cdot\lambda_b}{\Delta y}}(T_k - T_{a,b}) + \frac{\dot{q}_{IR,b}}{kA} - \frac{\dot{q}_{v,b,a}}{A} + \frac{\dot{m}_{v,n,k}}{A}\Delta h_v\{T_k\} \tag{2 d}$$

The movements of moisture in a hygroscopic material depend on the way that water is bound in the material. If the water is bound on the surface of a material, dynamic mass transfer mechanisms depend on the hygroscopicity of the material. An equation 3 demonstrates how much the partial pressure of water vapour in a hygroscopic material differs from the partial pressure of water vapour in saturated air. This difference is generally used for representing the hygroscopic behaviour of a material.

$$\varphi = p_v''/p_v' \tag{3}$$

In the process of calculating the amount of water transferred from a coating along with dynamic diffusion, an equation 4 will be applied. Vapour diffusion can be regarded as a determinant mass transfer mechanism in a hygroscopic material, if the material has a low moisture content. In paper, the moisture content must be less than 0.2 - 0.3 kg $H_2O$/kgbd, depending on a grade of paper, for this mass transfer mechanism to be a determinant. Generally, in the process of coating a paper, the base paper has a moisture content which is substantially less than 0.2 kg $H_2O$/kgbd, and the base paper maintains its moisture below this content during a drying process. Further information about the equation, for example about the reason why it is here shown in its linear form, can be found in literature [Berg 1999, Berg et al. 2001].

$$\frac{\dot{m}_v}{A} = \beta'_{eff}\frac{M_v}{RT}\left(p'_{v0}\varphi_0 - p'_{v1}\varphi_1\right) \tag{4}$$

$$\beta'_{eff} = \varepsilon\psi\eta\,\frac{D_{va}}{\Delta Y} \tag{5}$$

$$\beta'_{eff} = \frac{D_{va}}{\dfrac{\Delta Y_0}{\varepsilon\psi\eta_0} + \dfrac{\Delta Y_1}{\varepsilon\psi\eta_1}} \tag{6}$$

Equations 5 and 6 express what the mass transfer coefficient consists of. The coefficient set $\varepsilon\psi\eta$ takes generally the form of

$$\frac{D_{eff}}{D_v}$$

,i.e. the set $\varepsilon\psi\eta$ can be referred to as an effective diffusion coefficient for the discussed material. Equation 6 shows how two materials are connected in series regarding mass transfer, which two materials could be for example a coating and a base paper. Fig. 9 illustrates how the measured and calculated data is matched together. The Figure also shows how the contributions of base paper and coating to the transfer of water from coating to base paper can be distinguished from each other. The mathematically measured and calculated data can be matched by means of equation 7.

$$\min\left[\beta'_{eff}\,\frac{M_v}{RT}\left(p'_{v0}\varphi_0 - p'_{v1}\varphi_1\right) - \left(\frac{\dot{m}}{A}\right)_{\exp}\right]^2 \;\rightarrow\; \varepsilon\psi\eta_0 \;\text{and}\; \varepsilon\psi\eta_1 \qquad (7)$$

After being matched to measuring data, the diffusion coefficients can be exploited in terms of working out situations, wherein conditions are substantially different from those experienced during measurements. For example, it is possible to calculate product-specifically how a coated paper behaves in the drying section of a paper coating machine. Respective calculations have been compared with measured data from a pilot coating machine in Fig. 10. The diffusion coefficients applied as described above contain also the inaccuracy of a mathematical model and, for example, the inaccuracy of hygroscopicity, equation 3.

Fig. 9 illustrates by way of triangles the amount of water measured with a method of the invention and absorbed from a coating mix into a coated paper, as a function of time at the temperature of 23°C. These points are used as a basis for working out diffusion coefficients, the amount of water absorbed at the temperature of 23°C calculated thereby being represented by a thicker line. The diffusion coefficients have been used to calculate the amount of water absorbed at the temperature of 60°C, which is represented by a thinner line. The experimentally measured amount of water absorbed at the temperature of 60°C is represented by rhombi. In the caption of the Figure, z represents moisture content $kgH_2O/kgbd$.

Fig. 10 demonstrates information calculated with a method according to the invention for a pilot-scale paper coating machine. Temperatures calculated for the reverse side of paper and moisture calculated for paper are represented by a thicker line, and temperatures and moisture calculated for a coating are represented by a thinner line. The measured temperatures are represented by rhombi and the measured moistures by quadratures.

It is obvious for a person skilled in the art that the invention is not limited to just the above-described examples, but the invention may take various forms within the scope of the appended claims. The method can be automated, whereby the inventive system involves supplying paper in one location and a coating mix in another. This would be followed by an automatic delivery of measuring results. A viscosity measurement, or a sort of runnability measurement, for a coating is implementable, at least if the applied coating method is bar coating or blade coating. Temperature measurements on the coating and reverse side of paper can be effected for example by means of a thermocouple, by way of IR measurements, or conductively. There must be a possibility of adjusting the condition of air, its temperature and humidity, around the measuring apparatus. In practice, this is indeed possible in every paper laboratory. Preferably, a base paper and a coating can be heated, such that the measurements can be performed at two different temperatures, for example 23°C and 60°C. The number of sample knives can be increased for making a number of measurements in a single run. Sample knives can be arranged one after the other and/or a number of sample knives side by side. If several sample knives with various scraping depths are set in succession, it is possible to determine the moisture gradient in a coating layer.

**Literature**

Berg C-G., "Heat and Mass Transfer in Turbulent Moist Air Drying Processes", Thesis, Åbo Akademi University, 1999

Berg C-G, Rajala P, and Solin R., "An Experimental Evaluation of the Liquid Movement in the Paper Coating Process", 1st Nordic Drying Conference 2001, Trondheim, Norway, 2001

Heikkilä P., "A Study on the Drying Process of Pigment Coated Paper Webs", Thesis, Åbo Akademi University, 1993

Letzelter P., "Studier over avvattningen av en bestrykningssmet i en bladbestrykare", Licentiate work, Institute for Paper Chemistry, Åbo Akademi University, 1993

Kirstilä V., "Experimentell identifiering av bestrykninsmetens avvattning vid bestrykning papper", Graduation work, Process Design Laboratory, Åbo Akademi University, 2000

Abbreviations

| | | |
|---|---|---|
| $A$ | $m^2$ | area |
| $m$ | kg, kgbd | mass |
| $B$ | m | web width |
| $c, c_p$ | kJ/kgK | specific heat |
| $C, C_k$ | - | constant |
| $\dot{m}$ | kg/s | mass flow |
| $n$ | mol | mole |
| $l$ | m | length |
| $p, \Delta p_E$ | kPa | pressure, ext. pressure diff. |
| $q$ | kW | heat flow |
| $y, \Delta y$ | m | length feature, distance |
| $Y$ | $kgH_2O/kgtot$ | moisture ratio |
| $r$ | m | radius |
| $S_m$ | J/molK | molar entropy |
| $T, t$ | K, °C | temperature |
| $U_m$ | J/mol | molar internal heat |
| $x$ | $kgH_2O/kgda$ | air humidity |
| $w$ | m/s | speed |
| $V_m$ | m3/mol | molar volume |
| $v$ | $m^3/kg$ | specific volume |
| $R$ | J/molK | gas constant |
| $l_s$ | J/kg | sorption heat |
| $h_0, \Delta h_v$ | kJ/kg | specific enthalpy, evaporation heat |
| | | *Greek letters* |
| $\alpha$ | $W/m^2K$ | heat transfer coefficient |
| $\beta, \beta'$ | $W/m^2K$ | mass transfer coefficient |
| $\lambda$ | W/mK | thermal conductivity |
| $\theta$ | ° | contact angle |
| $\varphi$ | - | relative humidity |
| $\mu$ | J/kg | chemical potential |
| $\eta$ | kg/ms | dynamic viscosity |
| $\rho$ | $kg/m^3$ | density |
| $\tau$ | s | time |
| $y$ | N/m | surface tension |
| | | *Lower and upper indices* |
| $a$ | | air |
| $b$ | | base |
| $c$ | | coating |
| $e$ | | evaporation |
| $n, k$ | | continuous indexing, last line number |
| $v$ | | vapour |
| $w$ | | water |
| $l$ | | liquid |

(continued)

| tot<br>'''<br>' | | total amount<br>physical properties close to surface |
| | | *Other than SI abbreviations* |
| da, db, exp | | dry air, through dry material, experimental |

**Claims**

1. A method for determining quantities of a coating process, in which coating process a coating material is coated with a coating device (5) on a sheet- or web-like material (2), said sheet- or web-like material (2) being known for one or more quantity B and said coating material being known for one or more quantity C, said method comprising

   - moving the sheet- or web-like material (2) and the coating device (5) relative to each other in a direction of application (A),
   - applying one or more coating materials in the direction of application (A) on the sheet- or web-like material (2) for producing a coated material,

   **characterized in that** the method further comprises

   - using one or more sample knives (7) for scraping the coated material essentially in the same direction of application (A) for picking up a sample (11) of the coated material onto the sample knife (7) after a time T has lapsed from the application of the coating,
   - measuring one or more scraped-off samples (11) for one or more quantities D, and
   - measuring a scraped coated material (10) for one or more quantities E.

2. A method according to claim 1, **characterized in that** it further comprises calculating a desired coating process quantity or quantities by exploiting at least some of said quantities B, C, D and E.

3. A method according to claim 1 or 2, **characterized in that** a computer program is run on a computer, the program comprising a program code which is adapted to calculate a desired coating process quantity or quantities by exploiting at least some of said quantities B, C, D and E.

4. A method according to any of the preceding claims, **characterized in that** the material (2) to be coated is paper or board.

5. A method according to any of the preceding claims, **characterized in that** the scraping is followed by drying substantially all liquid off from the sample (11), and that the dried sample is weighed.

6. A method according to any of the preceding claims, **characterized in that** the quantities B, C, D and E are masses, such as a grammage of paper.

7. A method according to claim 6, **characterized in that** the measured masses are used as a basis for calculating a dry content for the sheet- or web-like material (2), the coating material and/or the coated material.

8. A method according to any of the preceding claims, **characterized in that** a dry content for the sheet- or web-like material (2), the coating material and/or the coated material is measured conductively with a laboratory-scale device, and preferably on-line.

9. A method according to any of the preceding claims, **characterized in that** the coated material is scraped for more than one samples (11) after times $T_1$, $T_2$,...$T_n$ have lapsed from the application of coating, the values measured from said samples (11) being used as a basis for calculating a change in the value of a desired quantity as a function of time lapsed from the application.

10. A method according to claim 9, **characterized in that** two or more samples (11) are picked up from the coated material at different depths.

**11.** A method for controlling a drying process, said method comprising at least the following steps

    a) determining quantities of a coating process in accordance with any of claims 1-10,
    b) using a first mathematical model to calculate diffusion coefficients for a material (2) to be coated and a coating material in the coating process by making use of measuring data obtained from step a),
    c) using a second mathematical model to calculate a drying process in conditions differing from those of step a), such as in an industrial coating process,
    d) controlling a drying process, such as an industrial coating process, on the basis of results obtained from step b) and/or c).

**12.** A system for determining quantities of a coating process, in which coating process a sheet- or web-like material (2) Is coated with a coating device (5), said system comprising at least

    **-**    a foundation (1) for the sheet- or web-like material (2),
    **-**    a coating device (5),
    **-**    guide elements (6a, 6b), along which the coating device (5) is adapted to be movable relative to the foundation (1) essentially in the same direction, i.e. in a direction of application (A),

    **characterized in that** the system further comprises a doctor device, comprising at least a frame element (8) and at least one sample knife (7) arranged thereto, said doctor device (7, 8) being adapted to be movable along the guide elements (6a, 6b) relative to the foundation (1) in the direction of application (A).

**13.** A system according to claim 12, **characterized in that** the sample knife (7) is removably arranged to the frame element (8) for investigating a sample (11) adhered to the sample knife (7).

**14.** A system according to claim 12 or 13, **characterized in that** the doctor device (7, 8) is arranged in a fixed connection with the coating device (5).

**15.** A system according to claim 12, 13 or 14, **characterized in that** the doctor device (7, 8) is provided with a sample knife elevating means (8) for controlling thereby the distance of the sample knife (7) from the foundation (1).

**16.** A system according to claim 12, 13, 14 or 15, **characterized in that** it further comprises a computer or the like for controlling functions of the system and/or for calculating desired quantities.

**17.** A computer program product, which is stored in a computer element readable by a computer, **characterized in that** it comprises a program code element for making a computer perform at least steps b) and c) of claim 11, when said computer program product is run in the computer.

**18.** A computer program product according to in claim 17, **characterized in that** it further comprises a program code element for making a computer perform step d) of claim 11.

EP 1 154 272 A2

Fig.3

Fig.4

Fig.5

Fig.2

Fig.1

12

**Step a)**
Drying measurements for coating and material to be
coated are performed with a method according
to the invention

**Step b)**
A mathematical model, which is based on fundamental
equations of thermodynamics, is used to determine diffusion
coefficients both for material to be coated and for
coating material by means of measuring data
obtained from step a)

**Step c)**
Diffusion coefficients obtained from step b) are used
in the process of calculating the drying of coating
and material to be coated in conditions substantially
different from those existing during measurements in step a)

**Step d)**
Results obtained from step b) and/or c) are used
for controlling industrial drying process

Fig. 6

$\dot{q}_{a,c}$

$\dot{m}_{v,c}/A$

$T_a$ $\alpha_{a,c}$

$\dot{q}_{IR,c}$ · $T_c$ · $Y_c$ $\dot{m}_{w,c,b}/A$ — Coating (c)

$\dot{q}_{IR,b,0}$ · $T_0$ · $Y_0$ $\dot{m}_{v,0,1}/A$

$\dot{q}_{IR,b,1}$ · $T_1$ · $Y_1$ $\dot{m}_{v,1,2}/A$

— Material to be coated

such as e.g. base paper (b)

$\Delta y$ $\dot{m}_{v,1-k,k}/A$

$\dot{q}_{IR,b,k}$ · $T_k$ · $Y_k$

$T_a$ $\alpha_{a,b}$

$\dot{q}_{a,b}$ $\dot{m}_{v,b}/A$

Fig. 7

EP 1 154 272 A2

Fig. 8

Measured and calculated data from laboratory tests

Fig. 9

Simulated and measured data from pilot machine

Fig. 10